# EUROPEAN PATENT SPECIFICATION

(11) **EP 3 062 781 B1**
(45) Date of publication and mention of the grant of the patent: **06.12.2017**
(21) Application number: 14784023.5
(22) Date of filing: 07.10.2014
(51) Int. Cl.: A61K 31/045, A61K 8/34, A61K 47/10, A61K 9/00, A61Q 5/00, A61P 17/00

(54) **METHOD FOR CONDITIONING THE SCALP TO RESIST DANDRUFF**
VERFAHREN ZUR KONDITIONIERUNG DER KOPFHAUT ZUM ENTGEGENWIRKEN VON SCHUPPEN
PROCÉDÉ POUR CONDITIONNER LE CUIR CHEVELU AFIN DE RÉSISTER AUX PELLICULES

(30) Priority: 28.10.2013 WO PCT/CN2013/086070; 13.12.2013 EP 13197155
(43) Date of publication of application: 07.09.2016
(73) Proprietor: UNILEVER N.V., 3013 AL Rotterdam (NL); Unilever PLC, London, Greater London EC4Y 0DY (GB)
(72) Inventor: BIAN, Xiaoying, Shanghai 200335 (CN); HARDING, Clive Roderick, Bebington Merseyside CH63 3JW (GB); LITTLE, Christopher John, Liverpool Merseyside L3 4AB (GB); NIRULA, Ritu, Shanghai 200335 (CN); TURNER, Graham Andrew, Wirral Merseyside CH63 3JW (GB); WELCH, Susan, Wirral Merseyside CH63 3JW (GB)
(74) Representative: Chisem, Janet
(86) International application number: PCT/EP2014/071404
(87) International publication number: WO 2015/062821

(56) References cited:
- EP-A1- 0 461 593
- JP-A- 2011 256 114
- US-A1- 2002 168 327
- US-A1- 2009 274 642
- US-A1- 2012 190 753
- US-B1- 7 001 594
- FLUHR J.W. ET AL: "Glycerol and the skin: holistic approach to its origin and functions", BRITISH JOURNAL OF DERMATOLOGY, vol. 159, no. 1, July 2008 (2008-07), pages 23-34, XP055114643, ISSN: 0007-0963, DOI: 10.1111/j.1365-2133.2008.08643.x
- LODEN M ET AL: "A DOUBLE-BLIND STUDY COMPARING THE EFFECT OF GLYCERIN AND UREA ON DRY, ECZEMATOUS SKIN IN ATOPIC PATIENTS", ACTA DERMATO-VENEREOLOGICA, TAYLOR & FRANCIS LTD, UNITED KINGDOM, vol. 82, no. 1, January 2002 (2002-01), pages 45-47, XP001121921, ISSN: 0001-5555, DOI: 10.1080/000155502753600885
- TURNER G. A. ET AL: "Stratum corneum dysfunction in dandruff", INTERNATIONAL JOURNAL OF COSMETIC SCIENCE, vol. 34, no. 4, 17 May 2012 (2012-05-17), pages 298-306, XP055114488, ISSN: 0142-5463, DOI: 10.1111/j.1468-2494.2012.00723.x cited in the application

## Description

### TECHNICAL FIELD OF THE INVENTION

The present invention relates to the treatment and/or prevention of dandruff and symptoms of dandruff. In particular the invention relates to glycerol for use in conditioning the scalp to resist dandruff.

### BACKGROUND TO THE INVENTION

It is widely believed that *Malassezia* yeasts, such as *Malassezia furfur,* are the main cause of dandruff. However, it is unclear why some people suffer from this condition while others do not. What is known is that increasing the level of *Malassezia* on the scalp does not automatically lead to dandruff. This suggests that *Malassezia* is necessary but not sufficient to cause the condition.
The main, if not only, intervention strategy used on the market currently for the treatment of dandruff is the topical application of anti-fungals such as zinc pyrithione (ZPTO), octopirox, climbazole and ketoconazole which are normally delivered from a shampoo. These antifungal agents remove (or at least reduce the level of) the *Malassezia* from the scalp, and provide moderately effective treatment of the dandruff condition. Shampoos contain cleansing surfactants such as sodium lauryl sulfate (SLS) or sodium laureth sulfate (SLES).
International patent application published as WO 02/067880 (Unilever) suggests an alternative approach to the treatment of dandruff. In particular WO 02/067880 discloses use of a lipophilic agent or a lipid precursor and/or promoter in the manufacture of a composition for treating and/or preventing dandruff by strengthening the scalp.
While the approach suggested in WO 02/067880 may be effective in ameliorating dandruff, lipophilic agents are not always suitable for topical application to the scalp especially under certain circumstances for consumers who are concerned about oiliness or greasiness of scalp and hair.

A recent publication (G.A. Turner, M. Hoptroff and C.B. Harding, "Stratum Corneum Dysfunction in Dandruff", International Journal of Cosmetic Science, 2012, 34, pp. 298-306) highlighted that shampooing with harsh surfactants will undermine the efficacy of conventional cosmetic antidandruff treatments by repeatedly damaging the scalp barrier. The strategies suggested to combat this effect include the use of mild surfactants. However mild surfactant systems can under some circumstances be expensive and/or show inferior cleaning performance compared to conventional anionic surfactant systems and so may not be suitable for all dandruff sufferers, especially those with greasy hair who are looking for deep cleansing.

The present inventors have surprisingly found that by treating the scalp with glycerol, the condition of the scalp barrier can be improved to such an extent that the scalp barrier can resist damage by even harsh surfactants. As a result even after the glycerol is washed away, the symptoms of dandruff are prevented from returning (or their progression is at least slowed) for a prolonged period.

The use of glycerol in compositions which comprise an antifungal agent for the treatment of dandruff is known. For example, US 7,001,594 (Peffly et al.) discloses topical leave-on cosmetic compositions, including packaged leave-on compositions, for direct application to the scalp, comprising a) from about 40 percent to about 99 percent by weight of a volatile liquid, b) from about 0.005 percent to about 20 percent by weight of a skin active agent, and c) from about 0.1 percent to about 20 percent by weight of a moisturizing material, preferably a liquid humectant, such as glycerin. The composition is allegedly effective at controlling dandruff. However the anti-dandruff efficacy is said to result through improved deposition of anti-dandruff actives such as ZPTO (present as the "skin active agent").

JP2011 256114 discloses lotions for application to the scalp, comprising polysaccharides, trihydric to hexahydric alcohols, which may be glycerol or diglycerol, and water for the successful treatment of dandruff and itching.

The present inventors have found that treating the scalp with glycerol can ameliorate the symptoms of dandruff, even in the absence of anti-fungal agents. Thus the present invention aims to provide a treatment for dandruff which utilizes glycerol to combat dandruff.

### SUMMARY OF THE INVENTION

In a first aspect the present invention provides glycerol for use in conditioning the scalp against the onset, return or worsening of symptoms associated with dandruff.

The invention may also be described in one or more of the following aspects:
- Glycerol as a medicament for use in conditioning the scalp against the onset, return or worsening of symptoms associated with dandruff.
- Non-therapeutic use of glycerol for conditioning the scalp against the onset, return orworsening of symptoms associated with dandruff.
- A non-therapeutic method for conditioning the scalp against the onset, return or worsening of symptoms associated with dandruff, the method comprising applying glycerol to the scalp of an individual, in an amount effective to improve the condition of the scalp barrier of the individual.

All other aspects of the present invention will more readily become apparent upon considering the detailed description and examples which follow.

### DETAILED DESCRIPTION

The glycerol for use in the present invention is variously known as glycerine, glycerin, and propane-1,2,3-triol.

Glycerol has now been found to condition the scalp against the onset, return or worsening of symptoms associated with dandruff. Without wishing to be bound by theory, the present inventors believe this improvement in the scalp condition is a result of strengthening of the scalp, possibly owing to enhanced epithelial differentiation in the scalp and/or an increased quality of the stratum corneum. Thus, according to a preferred aspect, the present invention provides for glycerol for use in conditioning the scalp against the onset, return or worsening of symptoms associated with dandruff. Such conditioning is preferably non-therapeutic, more preferably cosmetic.
By the term "strengthen the scalp", and related terms used herein, we mean that the resistance of the scalp to penetration by moisture is increased i.e., the water permeability barrier of the scalp is improved and/or strengthened. The loss of water through the stratum corneum may also be inhibited. Alternative descriptions for the strengthening of the scalp barrier, which are encompassed by the term as used herein, include, but are not limited to, one or more of the following: scalp nourishment, restoring the scalp barrier, strengthening the scalp, strengthening scalp resistance/defense, helping scalp regenerate, rebuilding the scalp, making smoother scalp skin, soothing the scalp, replenishing the scalp from within, building the scalp, building the scalp from within, building the scalp barrier from within, revitalising the scalp, moisturising the scalp, fully hydrating the scalp, activating the scalp's natural protection layer, awakening scalp's natural protection layer and similar terms.
By strengthening the scalp, the scalp is in a better condition to resist the action of irritants such as *Malassezia* and/or microbial metabolites. Additionally or alternatively the scalp may be in a better condition to resist degradation by harsh surfactants.
Thus in a preferred embodiment the scalp is conditioned against the onset, return or worsening of symptoms associated with dandruff following contact of the scalp with cleansing surfactant, especially anionic cleansing surfactant.
Examples of anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.
Harsh anionic cleansing surfactants that are widely used are those selected from sodium lauryl sulphate (=SLS) and sodium lauryl ether sulphate(n)EO (=SLES), (where n is from 1 to 3); more preferably sodium lauryl ether sulphate(n)EO, (where n is from 1 to 3); most preferably sodium lauryl ether sulphate(n)EO where n=1.
Preferably the scalp is contacted with shampoo composition comprising cleansing surfactant in an amount from 1.5 wt% to 25 wt% of the shampoo composition, more preferably from 3 wt% to 19 wt%, most preferably from 6 wt% to 17 wt% of the shampoo composition.
The cleansing surfactant may be applied to the scalp of the individual after and/or simultaneously with application of the glycerol to the scalp of the individual. When applied simultaneously, the composition comprising glycerol may, for example, also comprise the cleansing surfactant. For example, the glycerol and cleansing surfactant may be applied as components of the same shampoo composition.
In a preferred embodiment, the cleansing surfactant is applied to the scalp of the individual after application of the glycerol to the scalp of the individual. Thus it is preferred that the composition comprising glycerol is substantially free from anionic cleansing surfactant. More preferably the composition comprising glycerol comprises less than 1 % anionic cleansing surfactant by weight of the composition, even more preferably less than 0.5%, more preferably still less than 0.1% and most preferably from 0 to 0.01%.
Topically applying glycerol to the scalp is found not only to combat dandruff during the treatment period (i.e., the period in which the glycerol is applied to scalp of an individual at least once per week, more preferably at least twice per week and most preferably from three to seven times per week), but also into the regression phase when treatment is ceased. Thus the symptoms associated with dandruff are preferably reduced or prevented from returning for a regression period of at least 3 days following conditioning the scalp, more preferably for a regression period of at least 1 week following conditioning the scalp, and most preferably for a regression period of from 1 to 4 weeks.
Surprisingly this long-lasting effect may be maintained even if the scalp is treated with cleansing surfactant (e.g., by shampooing) throughout the regression phase. For example, the scalp may be contacted with cleansing surfactant at least twice during the regression period, more preferably at least three times and most preferably from four to ten times.
The glycerol may be used in substantially pure form. However in a preferred embodiment the glycerol is used in the form of a hair treatment composition, such as a shampoo, conditioner or leave-on treatment. Typically, the glycerol is present in the composition in an amount of from 0.001% to 60% by weight, more preferably from 0.1% to about 40% by weight, more preferably still from 1 % to 30% by weight, most preferably from 2% to 20% by weight of the composition.
The ability of the glycerol to condition the scalp may be enhanced if it is used with skin active agents. Thus in one embodiment the composition comprises skin active agent.
Examples of skin active agents include lipophilic agent or a lipid precursor and/or promoter as described in WO 02/067880 A1. Especially preferred lipophilic agents are hydrocarbon oils, hydrocarbon waxes, fatty acid derivatives, cholesterol and derivatives thereof, di-and tri-glycerides, vegetable oils and derivatives thereof, liquid non-digestible oils, polyol esters, acetoglyceride esters, alkyl esters and alkenyl esters of fatty acids, lanolin and its derivatives, wax esters, beeswax and its derivatives, sterols, phospholipids, ceramides, neoceramides, pseudoceramides and mixtures thereof. Most preferred are vegetable oils, especially sunflower oil.

Other skin active agents include vitamins, provitamins, derivatives thereof, and mixtures thereof. Especially preferred are B-vitamins and provitamins thereof, especially Vitamin B₃, panthenol and mixtures thereof. As used herein "Vitamin B₃" includes not only niacin (nicotinic acid), but also its corresponding amide (nicotinamide also known as niacinamide), as well as other amides and esters of niacin. The preferred form of Vitamin B₃ is niacinamide.
The total amount of skin active in the composition is preferably from 0.0001 to 30% by weight of the composition, more preferably 0.001 to 20%, more preferably still 0.01 to 15%, even more preferably from 0.1 to 10% and most preferably from 1 to 7%.
The present inventors have found that glycerol is effective at combating dandruff even in the absence of antifungal agents. Thus in one embodiment the composition may contain relatively low amounts of antifungal agent. For example preferably the total amount of antifungal agent in the composition is less than 0.5% by weight of the composition, more preferably the total amount of antifungal agent in the composition is less than 0.3% by weight of the composition, even more preferably less than 0.1 %, and most preferably less than 0.05%. In a most preferred embodiment the composition is substantially free from antifungal agents, more preferably the total amount of antifungal agent in the composition is less than 0.01 % by weight of the composition, even more preferably less than 0.001 %, and most preferably from 0 to 0.0001 %.
Antifungal agents typically display a minimum inhibitory concentration of about 50 mg/ml or less against *Malassezia spp.*
If antifungal agents are present in the composition, preferred amounts range from 0.01 to 5% wt. of the composition, more preferably from 0.1 to 2.5% wt. of the composition.

Examples of antifungal agents include compounds selected from azole-based antifungal agents, octopirox, metal pyrithione salts, selenium disulfide, coal tar and mixtures thereof. The preferred azole-based antifungal agents are ketoconazole and climbazole. Preferred metal pyrithione salts are zinc, copper, silver and zirconium pyrithione. The most preferred is zinc pyrithione.
Preferably, the composition comprises at least 5% of water by weight of the composition, more preferably from 15 to 95%, even more preferably from 35 to 88%, still even more preferably from 45 to 82%, most preferably from 65 to 80% by weight of the total composition.

The composition may contain other ingredients for enhancing performance and/or consumer acceptability. Such ingredients include silicones, alcohols, cationic surfactants, cationic polymers, fragrance, dyes and pigments, pH adjusting agents, pearlescers or opacifiers, viscosity modifiers, and preservatives or antimicrobials. Each of these ingredients will be present in an amount effective to accomplish its purpose. Generally these optional ingredients are included individually at a level of up to 5% by weight of the total composition.

Compositions are primarily intended for topical application to at least a portion of the scalp and/or hair of an individual, either in rinse-off or leave-on compositions. Preferably the composition is applied to the scalp in an amount of at least 0.3 g, more preferably at leasty 0.5 g, more preferably still at least 1 g and most preferably from 2 to 10 g.
Except in the examples, or where otherwise explicitly indicated, all numbers in this description indicating amounts of material or conditions of reaction, physical properties of materials and/or use may optionally be understood as modified by the word "about".
All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

The following examples are provided to facilitate an understanding of the invention. The examples are not intended to limit the scope of the claims.

### EXAMPLES

### EXAMPLE 1

A clinical study was conducted to investigate the effect of treatment with glycerol on dandruff.

### Method

The test (Sample 1) and control (Sample A) lotions were formulated with the compositions given in Table 1.

**TABLE 1**

| **Component %w/w** | **Sample 1** | **Sample A** |
|---|---|---|
| Glycerol | 8.0 | --- |
| Hydroxyethylcellulose | 0.6 | 0.6 |
| Sodium Benzoate | 0.5 | 0.5 |
| Water | To 100 | To 100 |

A total of 24 self-perceived dandruff sufferers were recruited as panellists and had various levels of dandruff. A randomised double blind half-head design was used. Lotions were applied by dividing the head into two symmetrical halves and then further into quadrants. Panellists were asked not to wash their hair 48 hours before their first visit to the Study Centre. Lotions were applied on their scalp for 5 consecutive days during which panellists were asked not to wash their hair or apply any other product on their scalps or hair (the "treatment phase"). Trained personnel carried out lotion application. The dosage applied was 0.75 ml per quadrant, as the scalp has four quadrants and only one side i.e. two quadrants will have product applied, therefore the total applied to one half head will be 1.5 ml. Scalp assessments were conducted by experts every day before lotion application. After 5 days of lotion application panellists were allowed to wash their hair using their normal shampoo and return on day 8 for a final assessment (the "regression phase").

The expert scalp assessment was conducted as follows: The subject was seated under a bench magnifying light on a trolley base with a cool white fluorescent tube that was used to assess the hair and scalp condition. The expert scalp assessors were trained in using a well-defined quadrant assessment methodology that results in a numerical score (the Total Weighted Head Score or "TWHS") of the amount of scalp affected by a)'dryness/dandruff on the scalp', b)'loose flakes in the hair', c)'natural redness on the scalp' and/or d)'erythema on the scalp'. The assessor used a tail-ended comb to part the hair in order to see the scalp skin. For each subject a sterilised comb and grips and a clean pair of disposable gloves was used. This was to ensure that no active skin condition was passed from one subject to another, and also as a preventative measure in the unlikely event of the human head lice and/or eggs spreading from one infected subject to another.

### Results

Sample 1 delivered a significantly (P<0.05) better (lower) TWHS than Sample A on all days except for day 1. This illustrates that the sample containing glycerol (Sample 1) had an anti-dandruff effect that persisted not only through the treatment phase but also for at least the 3 days after washing in the regression phase.

### EXAMPLE 2

A clinical study was conducted to investigate the effect of treatment with a lotion containing glycerol and skin active agents on dandruff.

### Method

The test (Sample 2) and control (Sample B) lotions were formulated with the compositions given in Table 2.

**TABLE 2**

| **Component %w/w** | **Sample 2** | **Sample B** |
|---|---|---|
| Deionised Water | To 100 | To 100 |
| Cetyl Alcohol | 0.40 | --- |
| Glyceryl Stearate (GMS) | 0.70 | --- |
| Stearic Acid | 2.54 | --- |
| Glycol Stearate | 1.50 | --- |
| Glycerine | 10.00 | --- |
| Sunflower Seed Oil | 0.59 | --- |
| Isopropyl Palmitate | 0.60 | --- |
| Dimethicone | 0.33 | --- |
| Magnesium Aluminium Silicate | 0.20 | 0.20 |
| Carbomer 980 | 0.06 | 0.40 |
| Triethanolamine | 0.69 | 0.69 |
| Methyl/Propyl Parabens | 0.30 | 0.30 |
| Disodium EDTA | 0.05 | 0.05 |
| Phenoxyethanol | 0.40 | 0.40 |

A half-head, double-blind randomised study was used and was similar to that described in Example 1 except for the following:
- A total of 113 panellists participated.
- Total lotion applied per half head was 3 ml.
- Panellists washed their hair 3 times per week with a commercial beauty shampoo and were assessed 48 hours after each wash (i.e. just before the next wash).
- The treatment phase lasted a total of 8 weeks and the regression phase was monitored for a further 8 weeks.

### Results

The mean TWHS values are listed in Table 3.

**TABLE 3**

| **Time** | **TWHS Sample 2** | **TWHS Sample B** |
|---|---|---|
| Week 0 (Baseline) | 50.8 | 50.6 |
| Week 2 | 38.0 | 40.0 |
| Week 4 | 35.7 | 39.3 |
| Week 6 | 38.4 | 40.8 |
| Week 8 | 38.9 | 41.6 |
| Week 8 +2 days | 40.9 | 44.1 |
| Week 8 +4 days | 41.6 | 44.0 |
| Week 8 + 6 days | 42.0 | 44.4 |
| Week 10 | 47.5 | 48.3 |
| Week 12 | 47.0 | 47.1 |
| Week 16 | 44.6 | 44.8 |

Statistical analysis of the data at each time point showed that Sample 2 delivered a significantly (P<0.01) better anti-dandruff effect than Sample B at all time points in the test phase and maintained this for 1 week (3 shampoo washes) into the regression phase.

### EXAMPLE 3

A clinical study was conducted to investigate the effect of treatment with a lotion containing glycerol and a skin active agent (Niacinamide) on dandruff.

### Method

The test (Sample 3) and control (Sample C) lotions were formulated with the compositions given in Table 4.

**TABLE 4**

| **Component %w/w** | **Sample 3** | **Sample C** |
|---|---|---|
| Deionised Water | To 100 | To 100 |
| Acrylates/C 10-30 Alkyl Acrylate Crosspolymer | 0.40 | 0.40 |
| Sodium Hydroxide | 0.10 | 0.10 |
| Preservative | 0.11 | 0.11 |
| Disodium EDTA | 0.05 | 0.05 |
| Polyoxyethylene 7 lauryl alcohol | 1.00 | 1.00 |
| PPG-1 -PEG-9 Lauryl Glycol Ether | 3.00 | 3.00 |
| Decamethylcyclopentasiloxane | 0.48 | 0.48 |
| Dimethicone | 0.04 | 0.04 |
| Fragrance | 0.5 | 0.5 |
| Ethanol | 5.0 | 5.0 |
| Niacinamide | 5.0 | --- |
| Glycerol | 5.0 | --- |

A half-head, double-blind randomised study was used and was similar to that described in Example 1 except for the following:
- A total of 54 panellists participated.
- Total lotion applied per half head was 3 ml.
- Panellists washed their hair 3 times per week with a commercial beauty shampoo and were assessed 48 hours after a wash at baseline, mid treatment, end treatment, mid regression and end of regression.
- The treatment phase lasted a total of 4 weeks and the regression phase was monitored for a further 4 weeks.

### Results

The mean TWHS values are listed in Table 5.

**TABLE 5**

| **Time** | **TWHS Sample 3** | **TWHS Sample C** |
|---|---|---|
| Week 0 (Baseline) | 30.5 | 30.5 |
| Week 2 | 21.5 | 25 |
| Week 4 | 17.4 | 21.5 |
| Week 6 | 18.2 | 20.2 |
| Week 8 | 17.8 | 20.8 |

Statistical analysis of the data at each time point showed that the combination of glycerol and niacinamide when delivered from a leave-on lotion (Sample 3) significantly reduced the level of dandruff after 2 weeks and 4 weeks of treatment (p<0.0001) compared to the control lotion (Sample C). The reduction in dandruff from the glycerol/niacinamide lotion (Sample 3) compared to placebo lotion (Sample C) was also significant both 2 and 4 weeks into the regression phase (p<0.005).

### EXAMPLE 4

Example shampoo compositions for use in the invention are detailed in Table 6.

**TABLE 6**

| **Ingredient % wt.** | **Shampoo 1** | **Shampoo 2** |
|---|---|---|
| Sodium Laureth Sulphate | 16 | 14 |
| Cocoamidopropyl betaine | 2 | 1.6 |
| Zinc Pyrithione | --- | 1.0 |
| Zinc Sulfate | --- | 0.1 |
| Silicone | 3.0 | 2.0 |
| Glycerol | 5.0 | 2.0 |
| Acrylic Acid Polymer (Carbomer) | --- | 0.6 |
| Ethyleneglycol Distearate (EGDS) | 1.5 | --- |
| Guar Hydroxypropyl Trimonium Chloride | 0.1 | 0.2 |
| Salt, Preservatives and Perfumes | 1.0 | 1.5 |
| Water | To 100 | To 100 |

## Claims

1. Glycerol as a medicament for use in conditioning the scalp against the onset, return or worsening of symptoms associated with dandruff.

2. Non-therapeutic use of glycerol for conditioning the scalp against the onset, return or worsening of symptoms associated with dandruff.

3. A non-therapeutic method for conditioning the scalp against the onset, return or worsening of symptoms associated with dandruff, the method comprising applying glycerol to the scalp of an individual, in an amount effective to improve the condition of the scalp barrier of the individual.

4. Glycerol for use, use or method as claimed in any one of the preceding claims, wherein the glycerol is used in combination with Vitamin B₃.

5. Glycerol for use, use or method as claimed in any one of the preceding claims, wherein the scalp is conditioned against the onset, return or worsening of symptoms associated with dandruff following contact of the scalp with cleansing surfactant.

6. Glycerol for use, use or method as claimed in claim 5, wherein cleansing surfactant comprises anionic surfactant, preferably anionic surfactant selected from Sodium Laureth Sulphate (SLS), Sodium Lauryl Ether Sulphate (SLES) and mixtures thereof.

7. Glycerol for use, use or method as claimed in any one of claims 5 or 6, wherein the cleansing surfactant is applied to the scalp of the individual after and/or simultaneously with application of the glycerol to the scalp of the individual.

8. Glycerol for use, use or method as claimed in claim 7, wherein the cleansing surfactant is applied to the scalp of the individual after application of the glycerol to the scalp of the individual.

9. Glycerol for use, use or method as claimed in any one of the preceding claims, wherein the symptoms associated with dandruff are reduced or prevented from returning for a regression period of at least 3 days following conditioning the scalp.

10. Glycerol for use, use or method as claimed in claim 9, wherein the symptoms associated with dandruff are reduced or prevented from returning for a regression period of at least 1 week following conditioning the scalp.

11. Glycerol for use, use or method as claimed in claim 9 or 10, wherein the scalp is contacted with cleansing surfactant at least twice during the regression period.

12. Glycerol for use, use or method as claimed in claim 11, wherein the scalp is contacted with cleansing surfactant at least three times during the regression period.

## Patentansprüche

1. Glycerin als Medikament zur Verwendung bei der Konditionierung der Kopfhaut gegen das Einsetzen, das Wiederauftreten oder das Verschlechtern von mit Schuppen verbundenen Symptomen.

2. Nicht-therapeutische Verwendung von Glycerin zur Konditionierung der Kopfhaut gegen das Einsetzen, das Wiederauftreten oder das Verschlechtern von mit Schuppen verbundenen Symptomen.

3. Nicht-therapeutisches Verfahren zur Konditionierung der Kopfhaut gegen das Einsetzen, das Wiederauftreten oder das Verschlechtern von mit Schuppen verbundenen Symptomen, wobei das Verfahren das Auftragen von Glycerin auf die Kopfhaut eines Individuums in einer Menge umfasst, die wirksam ist, den Zustand der Kopfhautbarriere des Individuums zu verbessern.

4. Glycerin zur Verwendung, Verwendung oder Verfahren, wie in irgendeinem der vorhergehenden Ansprüche beansprucht, wobei das Glycerin in Kombination mit Vitamin B₃ verwendet wird.

5. Glycerin zur Verwendung, Verwendung oder Verfahren, wie in irgendeinem der vorhergehenden Ansprüche beansprucht, wobei die Kopfhaut gegen das Einsetzen, das Wiederauftreten oder das Verschlechtern von mit Schuppen verbundenen Symptomen konditioniert wird, dem Kontakt der Kopfhaut mit Reinigungstensid folgend.

6. Glycerin zur Verwendung, Verwendung oder Verfahren, wie im Anspruch 5 beansprucht, wobei das Reinigungstensid anionisches Tensid, vorzugsweise anionisches Tensid, ausgewählt aus Natriumlaurethsulfat (SLS), Natriumlaurylethersulfat (SLES) und Mischungen davon, umfasst.

7. Glycerin zur Verwendung, Verwendung oder Verfahren, wie in irgendeinem der Ansprüche 5 oder 6 beansprucht, wobei das Reinigungstensid auf die Kopfhaut des Individuums nach und/oder gleichzeitig mit dem Aufbringen des Glycerins auf die Kopfhaut des Individuums aufgetragen wird.

8. Glycerin zur Verwendung, Verwendung oder Verfahren, wie im Anspruch 7 beansprucht, wobei das Reinigungstensid auf die Kopfhaut des Individuums nach Aufbringen des Glycerins auf die Kopfhaut des Individuums aufgetragen wird.

9. Glycerin zur Verwendung, Verwendung oder Verfahren, wie in irgendeinem der vorhergehenden Ansprüche beansprucht, wobei die mit Schuppen verbundenen Symptome für eine Regressionsperiode von mindestens drei Tagen, folgend der Konditionierung der Kopfhaut, vermindert oder vom Wiederauftreten abgehalten werden.

10. Glycerin zur Verwendung, Verwendung oder Verfahren, wie im Anspruch 9 beansprucht, wobei die mit Schuppen verbundenen Symptome für eine Regressionsperiode von mindestens einer Woche, folgend der Konditionierung der Kopfhaut, vermindert oder vom Wiederauftreten abgehalten werden.

11. Glycerin zur Verwendung, Verwendung oder Verfahren, wie im Anspruch 9 oder Anspruch 10 beansprucht, wobei die Kopfhaut mit dem Reinigungstensid während der Regressionsperiode mindestens zweimal in Kontakt gebracht wird.

12. Glycerin zur Verwendung, Verwendung oder Verfahren, wie im Anspruch 11 beansprucht, wobei die Kopfhaut mit dem Reinigungstensid während der Regressionsperiode mindestens dreimal in Kontakt gebracht wird.

## Revendications

1. Glycérol en tant que médicament pour utilisation dans le conditionnement du cuir chevelu contre l'apparition, le retour ou l'aggravation de symptômes associés aux pellicules.

2. Utilisation non thérapeutique de glycérol pour le conditionnement du cuir chevelu contre l'apparition, le retour ou l'aggravation de symptômes associés aux pellicules.

3. Procédé non thérapeutique pour le conditionnement du cuir chevelu contre l'apparition, le retour ou l'aggravation de symptômes associés aux pellicules, le procédé comprenant l'application de glycérol sur le cuir chevelu d'un individu, en une quantité efficace pour améliorer l'état de barrière du cuir chevelu de l'individu.

4. Glycérol pour utilisation, utilisation ou procédé selon l'une quelconque des revendications précédentes, dans lequel (laquelle) le glycérol est utilisé en association avec de la vitamine B₃.

5. Glycérol pour utilisation, utilisation ou procédé selon l'une quelconque des revendications précédentes, dans lequel (laquelle) le cuir chevelu est conditionné contre l'apparition, le retour ou l'aggravation de symptômes associés aux pellicules après le contact du cuir chevelu avec un tensioactif nettoyant.

6. Glycérol pour utilisation, utilisation ou procédé selon la revendication 5, dans lequel (laquelle) le tensioactif nettoyant comprend un tensioactif anionique, de préférence un tensioactif anionique choisi parmi le laureth sulfate de sodium (SLS), le sulfate d'éther laurylique de sodium (SLES) et les mélanges de ceux-ci.

7. Glycérol pour utilisation, utilisation ou procédé selon l'une quelconque des revendications 5 ou 6, dans lequel (laquelle) le tensioactif nettoyant est appliqué sur le cuir chevelu de l'individu après et/ou en même temps que l'application du glycérol sur cuir chevelu de l'individu.

8. Glycérol pour utilisation, utilisation ou procédé selon la revendication 7, dans lequel (laquelle) le tensioactif nettoyant est appliqué sur le cuir chevelu de l'individu après application du glycérol sur le cuir chevelu de l'individu.

9. Glycérol pour utilisation, utilisation ou procédé selon l'une quelconque des revendications précédentes, dans lequel (laquelle) les symptômes associés aux pellicules sont réduits ou empêchés de réapparaître pendant une période de régression d'au moins 3 jours après le conditionnement du cuir chevelu.

10. Glycérol pour utilisation, utilisation ou procédé selon la revendication 9, dans lequel (laquelle) les symptômes associés aux pellicules sont réduits ou empêchés de réapparaître pendant une période de régression d'au moins 1 semaine après le conditionnement du cuir chevelu.

11. Glycérol pour utilisation, utilisation ou procédé selon la revendication 9 ou 10, dans lequel (laquelle) le cuir chevelu est mis en contact avec un tensioactif nettoyant au moins deux fois pendant la période de régression.

12. Glycérol pour utilisation, utilisation ou procédé selon la revendication 11, dans lequel (laquelle) le cuir chevelu est mis en contact avec un tensioactif nettoyant au moins trois fois pendant la période de régression.
